(19) Europäisches Patentamt
European Patent Office
Office européen des brevets

(11) Publication number: **0 131 050**
**B1**

(12) **EUROPEAN PATENT SPECIFICATION**

(45) Date of publication of patent specification: **18.04.90**

(51) Int. Cl.$^5$: **A 61 K 31/14**

(21) Application number: **84900654.9**

(22) Date of filing: **05.01.84**

(86) International application number: **PCT/US84/00001**

(87) International publication number: **WO 84/02649 19.07.84 Gazette 84/17**

(54) **Use of a tetraethylammonium compound.**

(30) Priority: **10.01.83 US 456732**

(43) Date of publication of application: **16.01.85 Bulletin 85/03**

(45) Publication of the grant of the patent: **18.04.90 Bulletin 90/16**

(84) Designated Contracting States: **AT BE CH DE FR GB LI LU NL SE**

(56) References cited:
**US-A-2 295 504**
**US-A-2 666 009**
**US-A-4 262 007**

**J. M. Jones, Ed., Physicians, Desk Refernce to Pharmaceutical Specialties and Biologicals, 6th Edition, published 1952, by Medical Economics, Inc. (Rutherford, N.J.), see page 491 "Etamon Chloride TEAC."**

**The file contains technical information submitted after the application was filed and not included in this specification**

(73) Proprietor: **BALDONE, Joseph A.**
**1211 Royal Street**
**New Orleans, LA 70116 (US)**

(72) Inventor: **BALDONE, Joseph A.**
**1211 Royal Street**
**New Orleans, LA 70116 (US)**

(74) Representative: **Patentanwälte Grünecker, Kinkeldey, Stockmair & Partner**
**Maximilianstrasse 58**
**D-8000 München 22 (DE)**

**EP 0 131 050 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to the use of a tetraethylammonium compound for the manufacture of a medicament for the treatment of diseases caused by Herpes simplex (HSV) Types 1 and 2.

There are two main variants of Herpes simplex Virus (HSV), Types 1 and 2. Although they are capable of strong cross reaction in some assays, they can be differentiated by neutralization kinetics and, with greater accuracy, by restricted analysis of their purified deoxyribonucleic acid (DNA). Generally, infection by HSV Type 1 is associated with oral, facial and ocular lesions; infection by HSV Type 2 with genital and anal lesions.

Both HSV Type 1 and HSV Type 2 show a predilection for ectodermal tissues such as in their production of lesions in skin, oral cavity, vagina, conjunctiva and the nervous system. Venereal HSV is now epidemic in the U.S.A. Some twenty million persons are presently afflicted with this disease in this country. New cases and recurrences exceed 500,000 annually. HSV infections often cause blindness, neonatal deaths and encephalitis, and additionally result in huge economic losses to the nation and the world.

(A) Pathogenesis of HSV disease

An important characteristic of these viruses is their ability to persist in a latent, or quiescent, form in man and animals.

Initial or primary infections by HSV 1 and 2 are contracted through breaks in the mucus membrane where they replicate locally. From there, they spread to the regional lymph nodes and, occasionally, they can invade the bloodstream, producing viremia.

When the primary infection subsides, or recedes, the virus persists in a latent form in the sensory ganglia which innervate the site of primary infection. For ocular or oral infections, the viruses persist in the trigeminal ganglia. In genital infections, the viruses persist in the sacral ganglia.

Although the state of the viral genome during latency is not yet known, latency can be upset, resulting in viral multiplication. This produces the second form of the disease, which is the recurrent form. Recurrences usually occur at the primary sites. Such recurrent disease in humans can be induced by heat, cold, sunlight (ultraviolet light), hormonal and emotional disturbances, or by immunosuppressive agents.

The natural source and host for HSV Types 1 and 2 disease is man and the primary mode of transmission is by close personal contact.

(B) Epidemiology of HSV diseases

Epidemiological control of HSV Disease is poor because the majority of the population, up to 90%, has been exposed to the viruses. In the healthy carrier the viruses can be isolated in the tears, saliva, vaginal and other secretions even during the absence of overt disease.

Past treatments of the disease have been largely ineffective. To successfully stop the growth of a virus, an agent must selectively inhibit any of the viral specific functions such as (1) adsorption, (2) uncoating, (3) transcription, (4) protein synthesis, (5) nucleic acid replication, (6) maturation, and (7) release.

Among agents thus far licensed are: Idoxuridine (IDU) Cystosine Arabinoside (ARA-C), Adenine Arabinoside (ARA-A), Triflourothymidine (TFT), and Acyclovir. Interferon has also been tested for HSV treatment. All of these therapies interfere with viral and host cellular functions. Because of host cell toxicity the five chemicals (IDU, ARA-C and ARA-A, TFT, and Acyclovir) have been largely ineffective for systemic use in humans.

Until this invention there has been no drug shown to be capable of selective inhibition of viral function. The inventor has demonstrated that tetraethylammonium chloride (TEA) in the manner utilized does selectively inhibit viral function. The anti-viral properties of TEA toward HSV Types 1 and 2 is shown by the results in the detailed description of the invention given below, (appendix A). Similar properties are expected of the homologues of TEA.

The present invention intends the use of TEA. The TEA anion has the following structure:

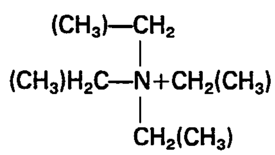

TEA is listed in the American Druggist's Blue Book (1974) Am. ed. as being sold by:

City Chemical Corp.
132 W. 22nd Street
New York, NY 10011

Description of a process for manufacture of TEA is given in US—A—2.653.156 which also notes generally the disinfectant properties of quaternary ammonium compounds of which TEA is one.

Similarly, US—A—4,165,373 contemplates the general use of quaternary ammonium compounds in a low foaming medium for external use as a general disinfecting agent.

US—A—2,689,814, similarly noted the germicidal effectiveness of TEA as well as fungicidal and

anesthetic properties of quaternary ammonium compounds. US—A—2,886,487 also describes the use of such quaternary ammonium compounds in topical application.

In the 1949 and 1957 edition of the J. M. Jones, Physician's Desk Reference to Pharmaceutical Specialties and Biologicals the product "Etamon Chloride", was reported to contain 0.1 g of tetraethylammonium chloride (quaternary ammonium compound) in each ml and described that said product "reversibly blocks transmission of the motor impulses of both the sympathetic and parasympathetic divisions of the autonomic nervous system. In conditions associated with vasospasm it causes vasodilation. Used in herpes zoster, postherpetic pain, causalgia, thromboangiitis obliterans (Buerger's disease), Raynaud's disease, thrombophlebitis, trenchfoot, and immersion foot. In the 1954 edition of Physicians' Desk Reference any claim with respect to herpes zoster was omitted. In the 1960 edition of the Physicians' Desk Reference the product was no longer listed, and subsequently the manufacture of the product was discontinued. It is further noted that this product contained as a preservative benzethonium chloride, which preservative in accordance with US—A—4,262,007 was effective in treating herpes zoster, etc.

In The Pharmacological Basis of Therapeutics (2nd Ed.) by Goodman and Gilman (1955; MacMillan) in a discussion of the therapeutic uses of TEA, TEA's role as a pain reliever was mentioned in relationship to inter alia herpes zoster as follows:— "In various types of causalgia and related painful posttraumatic states, herpes zoster, and chest pain caused by embolism, neoplasm, pleuritis, etc., TEA is of value in relieving pain, in a few instances permanently, and may be a useful diagnostic tool for selecting cases which might be relieved by sympathectomy".

Additional prior patents which may be of interest are US—A—2 295 504 and US—A—2 666 009.

The present invention contemplates the use of a tetraethylammonium compound manufacture of a medicament for the treatment of diseases caused by Herpes Simplex Virus (HSV) Types 1 and 2. The treatment involves administering the tetraethylammonium ion usually as TEA, to the infected host.

TEA is listed as a ganglionic blocking drug reversibly blocking both sympathetic and parasympathetic motor impulses.

Secondary effects include the lowering of blood pressure due to pronounced vasodilator action, mydriasis (pupil dilation) cycloplegia (which may cause temporary blurred vision), ptosis (lid drooping) and similar impairment of physical responses associated with its nerve impulse blocking action.

These and other advantages are obtained by administering TEA by injection or topical application, either before or after the virus is found present.

Dosages parenterally, that is for example intravenously and intramuscularly, vary when using TEA. The IV dose is preferably 200 to 500 mg not to exceed 7 mg per kg of body weight. The I.M. dose is preferably 1000 to 1200 mg not to exceed 20 mg per kg of body weight.

The LD-50 in rats is 2,630 mg per kg of body weight. The action of TEA does not modify conduction of nerve impulses but only blocks transmission of these impulses. This blocking action is fully reversible and treatment of excessive dosages is known in the art, such as the administration of acetylcholine.

Herpes lesions are manageable and self limiting in most cases but in many cases recurrences cause severe problems, for example, blindness through scarring of the cornea, encephalitis, neonatal deaths, etc.

It is known and accepted that the latent viruses reside in the ganglia and are reactivated to produce recurrent episodes of the disease. Inductions of recurrent episodes of the disease have been experimentally produced in animals by a variety of stimuli including physical manipulation of the sensory ganglia. In all cases of reactivation a change is seen in the reservoirs, the ganglia. It is therefore concluded that an agent which achieves host ganglionic blockage may have a profound effect on the recurrent diseases.

In individual doses it is advocated that unpreserved TEA be used. For multiple dose vials preservatives may be used. Exemplary preservatives are benzyl alcohol, butylparaben, chlorobutanol, metacresol, methylparaben, myristylgamma picolinium chloride, phenol, phenylmercuric nitrate, propylparaben and thimerosal.

Elsewhere in this disclosure is described in detail the antiviral activity of TEA. It is shown that it inhibits the in vitro replication of HSV Types 1 and 2. This antiviral activity was achieved when the compound was added before or after viral innoculation. Changes in cell morphology at high concentrations were totally reversible. It was also demonstrated that the activity of TEA did not impair cell metabolic processes. These discoveries indicate that this compound may be utilized as a selective and specific inhibitor of viral growth.

Additional advantages of the present invention are presented in the detailed description which follows.

Medication

The preferred embodiment includes the use of a tetraethylammonium compound in particular tetraethylammonium chloride for the manufacture of a medicament for the treatment of diseases caused by Herpes simplex 1 and 2 wherein the medicament is in the form of a typical, intravenous or intramuscular application.

Dosage parenterally, that is intravenously and intramuscularly, vary when using TEA. The intravenous dose is 200 to 500 mg not to exceed 7 mg per kg of body weight. The intramuscular dose is 1000 to 1200 mg not to exceed 20 mg per kg of body weight.

In individual doses it is advocated that unpreserved TEA be used. For multiple dose vials preservatives

may be used. Exemplary preservatives are benzyl alcohol, butylparaben, chlorobutanol, metacresol, methylparaben, myristylgamma picolinium chloride, phenol, phenylmercuric nitrate, propylparaben and thimerosal.

Experiments

In order to test the effectiveness of treating Herpes simplex virus with tetraethylammonium, in vitro tests were performed and verified by two additional duplicate experiments for reproducibility. The test safeguards, procedures and results given below show the effectiveness of the invention.

The overall aim of the experiments was to determine the antiviral activity of a tetraethylammonium compound, in particular tetraethylammonium chloride, hereafter referred to as TEA.

The determination of whether compound TEA could inhibit the in vitro replication of Herpes simplex virus 1 and 2, hereinafter referred to as HSV-1 and HSV-2, was sought.

I. Methods & materials

1) Cells and viruses

Monolayer cultures of Vero, RK-13 (rabbit kidney) and WISH (human amnion, Hayflick) cells were used in the study.

The cells were grown in Basal Minimal Media supplemented with 5% fetal calf serum, 1% glutamine, sodium bicarbonate and antibiotics. Cultures were maintained with the same media containing 2% fetal calf serum (maintenance media).

The F strain of HSV-1 was chosen because it is a well known prototype of HSV-1. It can be obtained from the American Type Culture Collection, 12301 Parklawn Drive, Rockville, MD., 20852. It is identified as ATCC-VR 733. For studies with Type 2, we used the G Strain (ATCC-VR 734) and also the 333 strain were used.

2) Plaque inhibition assay

Monolayer cultures of WISH cells were grown in 25 $cm^2$ plastic bottles of 6 well cluster dishes and were infected with 0.2 ml of the virus strain. They were allowed to absorb for 45 min at 37°C with gentle rocking of the cultures every 15 min.

After the absorption period, 1 ml of the drug dilution plus maintenance media were added to the cultures. The 25 $cm^2$ flashes received 1 ml of the drug dilutions plus 4 ml of maintenance media. Cluster dishes received 1 ml of drug dilution plus 3 ml of media. All cultures were prepared in duplicate.

3) Pre-incubation experiments (assays)

In these experiments the media was either decanted or gently aspirated from the flask, then 1 ml of drug dilution plus 1 ml of maintenance media was added. The cultures serving as virus controls or cell controls received 2 ml of maintenance media. After the pre-treatment period the media was again aspirated, and the cell cultures were infected as described above.

The cultures were incubated at 37°C for 36—48 h or until the virus control showed discrete visible plaques. When this occurred the cultures were fixed and stained with crystal violet, and the plaques counted under the microscope. The number of plaques in each treated culture was compared with those of the controls. A mean plaque value was obtained for each duplicate culture. From this data the percentages of plaque inhibition were then calculated.

Two methods were used to test the drug's effectiveness in the suppression of viral growth. The drug was added after adsorption of the virus in one set of experiments; and prior to infection of the cell cultures in others.

In one instance the effect of the drug on a cell that has already been infected and the virus has already penetrated into the cell's cytoplasm was being measured. In the other experiment, the cell cultures were treated with the drug prior to the innoculation to determine if these cells then could be permissive or non-permissive to this virus. Plaque reduction assays give a quantitative measure of anti-viral activity and is reliable and objective.

4) Toxicity

For the toxicity experiments all 3 cells, RK-13, Vero cells, and WISH cells were used. In these experiments the determination of the effect of the drug on the cell for long periods of time was sought. To this end cultures were incubated with and without the drug. The time of exposure was from 1 to 7 h. Any change in the morphology of the cultures was noted.

After each period of incubation the cells were washed, and fresh media was put in. The viability was then determined by the dividing of these cultures and having them grow into two new cell cultures.

5) Drug dilution

All drug dilutions were made in maintenance media.

II. Results

1) Anti-viral activity

Experiment #1

The effect of TEA on monolayer cultures of Vero and WISH cells was determined. Confluent monolayer cultures on 25 $cm^2$ plastic bottles were incubated overnight at concentrations of 5, 2.5 and 1.25 mg/culture. No change in morphology was seen. At the end of the incubation period, the cultures were washed twice with sterile PBS (Dulbecco). Fresh media was added to each of the cultures which were then incubated for an additional 24 h. There apparently was no difference in the growth or general appearance between the treated cultures and the untreated controls.

Although there was no appreciable difference between Vero and WISH, the latter, the human cell line, was chosen for use.

Experiment #2

Vero:

Confluent monolayer cultures of Vero cells were infected with the F strain of HSV-1. After absorption, the cultures received 1 ml of TEA at a concentration of 2.5 mg/ml plus 4 ml of maintenance media. Control cultures received no drug. Cultures were strained at the appropriate time and the plaques were counted. Drug treated cultures had an average of 75.5 plaques and the control cultures had an average of 488.5 plaques. The percent of plaque inhibition was therefore 84%. The size of the plaques in the treated cell cultures were appreciably smaller than those present in the control cultures.

Experiment 3

Based on the results obtained in the preceding experiment we sought to define the antiviral activity of TEA in WISH cells. The dilutions of TEA were 10, 5, 2.5 and 0.65 mg/ml. The HSV-1 (F) strain of virus was used. After adsorption (45 min 1 ml of drug dilution plus 3 ml of media went into each of two wells.

In another plate duplicate wells received 1 ml of drug dilution plus 1 ml of media and were incubated for 3 h. Then the media was aspirated, the cultures infected, and fresh media added. They were incubated at 37°C until discrete plaques were observed in the control wells. The findings from this experiment (Table I) are as follows:

1) Antiviral activity was seen in all the dilutions tested.

2) There was no strict correlation between viral inhibition and dosage; that is to say drug concentrations of 5 and 2.5 mg/ml which differ by a factor of 2 gave similar degrees of inhibition (45 & 46%).

3) Pre-incubation of the cell monolayer with the drug (10 mg), prior to innoculation, inhibited 71% of the plaques. Pre-incubation with 5 and 2.5 mg gave 22 and 10% inhibition which is not considered significant.

Pre-incubation rendered the cells non-permissive and different drug dilutions gave similar percent inhibition. From this it is concluded that the mode of action of this drug is other than a viral metabolic process.

Experiment #4

This experiment uses the same protocol of the preceding one, except that the drug concentrations were increased by a factor of ten. The data is shown in Table II.

The previous findings were confirmed. Pre-incubation of the cells with several dilutions gave comparable degrees of plaque inhibition. In the other cultures in which the drug was added after the adsorption period, the following was found: 100 mg per culture were toxic and the cells had lifted off the plate. The next concentrations, 50 and 25 mg per culture, did not exhibit any plaques, but the cell layer was "thin". The word "thin" means that the cells are still attached to the plastic, but the monolayer is not confluent, and there are spaces between the individual cells.

The 62% plaque-inhibition seen in the cultures containing 12.5 mg is consistent within the range of 56% plaque inhibition per 10 mg of drug found in the preceding experiment.

Those cultures that received the drug prior to innoculation gave relatively the same degree of protection (79—82%) regardless of concentration.

The percent inhibition found in the flasks that received the drug after virus adsorption could not be strictly compared with those cultures which received the drug prior to innoculation, because the volumes of fluid over the monolayers are different. This is very important in dealing with a membrane phenomenon.

Experiment #5

In this next experiment this phenomenon was investigated: All cultures received the same volume of 2 ml prior to innoculation and the same volume after innoculation.

Mock innoculated controls were also included, that is, these are cultures which were treated the same as the experimental flasks, but received no virus.

Findings:

TEA is a potent antiviral agent. It inhibits virus growth by rendering the cell non-permissive to the virus.

In these controlled experiments TEA action was observed to be dependent on both concentrations and duration of exposure to the drug.

Example:

50 mg/culture gives plaque-inhibition rates of:
24% at 2h pre-incubation
37.5% at 4h pre-incubation
95.7% at 7h pre-incubation.

Somewhat the same is seen with 25 mg/per culture, where (as shown in Table III) the percent inhibition rises from 17% to 96.5% with time.

Experiment #6

The effect of compound TEA was then tested against Herpes simplex type 2.

Table IV shows the results of one experiment. The percent inhibition of 56% with 50 mg/culture pre-incubation time is consistent with the results found with HSV-1. If any difference, HSV-2 (333) is more sensitive to the drug. The HSV-2 (G) strain was also tested, giving similar results.

III. Toxicity

The apparent toxicity that was described as "thin", is totally reversible. When the drug is removed and fresh media is added, the cell layer reverts to its normal non-treated appearance.

No change in morphology or cytopathic effect was seen in the cultures containing the drug nor those without the drug. There was no difference in the rate of growth of drug-tested cultures and those without drug. These observations indicate that the drug is not toxic to cells at the concentrations used.

TABLE I
Effects of compound of TEA in Herpes simplex virus replication HSV-1 (F)

| mg/cultures | % plaque inhibition |
|---|---|
| 10 | 56 |
| 5 | 45 |
| 2.5 | 46 |
| 1.25 | 39 |
| .62 | 30 |
| **3h pre-incubation** | |
| 10 | 71 |
| 5 | 22 |
| 2.5 | 10 |

TABLE II
Effect of compound TEA in Herpes simplex virus replication HSV-1 (F)

| mg/culture | % plaque inhibition[a] | 3h pre-incubation[b] |
|---|---|---|
| 100 | toxic* | 79 |
| 50 | thin* | 82 |
| 25 | thin* | 79 |
| 12.5 | 62 | 80 |
| 6.2 | 20 | — |

[a] Drug added after virus adsorption.
[b] Cultures pre-incubated with the drug 3h before infection.
* See text for explanation.

TABLE III
Effect of compound TEA on Herpes simplex virus replication HSV-1 (F)

| Pre-incubation time | mg/culture | % plaque inhibition |
|---|---|---|
| 2h | 100 | 68 |
| " | 50 | 24 |
| " | 25 | 17 |
| " | 12.5 | 18 |
| " | 6.2 | 0 |
| 4h | 100 | thin* |
| " | 50 | 63 |
| " | 25 | 37.5 |
| " | 12.5 | less than 20 |
| " | 6.25 | less than 20 |
| 7h | 100 | thin* |
| " | 50 | 95.7 |
| " | 25 | 96.5 |
| " | 12.5 | 76 |
| " | 6.25 | 50 |

* See text for explanation.

TABLE IV
Effect of compound of TEA on Herpes simplex virus replication HSV-2 (333) strain

| Pre-incubation time | mg/culture | % plaque inhibition |
|---|---|---|
| 2h | 50 | 56 |
| " | 25 | 42 |
| " | 12.5 | 25 |
| " | 6.25 | 0 |

Summary

1. Compound TEA inhibits the in-vitro replication of herpes simplex virus types 1 and 2.
2. The anti-viral effect of TEA is present whether it is added before or after viral innoculation.
3. When TEA was added before viral innoculation, it rendered the cells non-permissive to the virus and no plaques were formed. In these experiments, the anti-viral activity was time and dosage dependent.
4. Suppression of viral growth was evidenced by the reduced size and number of plaques.
5. These preliminary experiments suggest that the mode of action of TEA is not impairment of the cell metabolic process, but is rather related to changes in the cell membrane.
6. The observed toxicity or changes in the cell's morphology was totally reversible.

**Claims**

1. Use of a tetraethylammonium compound for the manufacture of a medicament for the treatment of diseases caused by Herpes Simplex Virus (HSV) Types 1 and 2.
2. Use according to claim 1, wherein the tetraethylammonium compound is tetraethylammonium chloride.
3. Use according to claim 1 or 2, wherein the medicament is in the form of a topical, intravenous or intramuscular application.
4. Use according to claim 1, wherein the transfer medium for intravenous or intramuscular injection contains of the order of 0.1 g of tetraethylammonium chloride per cubic ml of solution.
5. Use according to claim 4, wherein the dosage for intravenous injection is from 200 to 500 mg of tetraethylammonium chloride not to exceed 7 mg per kg of body weight of the recipient.
6. Use according to claim 4, wherein the dosage for intramuscular injection is from 1,000 to 1,200 mg of tetraethylammonium chloride not to exceed 20 mg per kg of body weight of the recipient.

**Patentansprüche**

1. Verwendung einer Tetraethylammoniumverbindung zur Herstellung eines Medikaments zur Behandlung von Krankheiten, die durch Herpes Simplex Virus (HSV)-Typ 1 und 2 verursacht werden.

2. Verwendung nach Anspruch 1, worin die Tetraethylammoniumverbindung Tetraethylammoniumchlorid ist.

3. Verwendung nach Anspruch 1 oder 2, worin das Medikament in Form einer örtlichen, intravenösen oder intramuskulären Anwendung ist.

4. Verwendung nach Anspruch 1, worin das Übertragungsmedium zur intravenösen oder intramuskulären Injektion etwa 0,1 g Tetraethylammoniumchlorid pro $ml^3$ Lösung enthält.

5. Verwendung nach Anspruch 4, worin die Dosierung zur intravenösen Injektion 200 bis 500 mg Tetraethylammoniumchlorid beträgt, wobei sie 7 mg pro kg Körpergewicht des Empfängers nicht übersteigt.

6. Verwendung nach Anspruch 4, worin die Dosierung zur intramuskulären Injektion 1000 bis 1200 mg Tetraethylammoniumchlorid beträgt, wobei sie 20 mg pro kg Körpergewicht des Empfängers nicht übersteigt.

**Revendications**

1. Utilisation d'un composé de tétraéthylammonium à la préparation d'un médicament pour le traitement des maladies provoquées par le virus de l'herpès (HSV) types 1 et 2.

2. Utilisation selon la revendication 1, dans laquelle le composé de tétraéthylammonium est le chlorure de tétraéthylammonium.

3. Utilisation selon la revendication 1 ou 2, dans laquelle le médicament est sous la forme d'une application locale, intra-veineuse ou intra-musculaire.

4. Utilisation selon la revendication 1, dans laquelle le milieu de transfert pour l'injection intra-veineuse ou intra-musculaire contient une quantité de l'ordre de 0,1 g de chlorure de tétraéthylammonium par millilitre cube de solution.

5. Utilisation selon la revendication 4, dans laquelle le dosage pour l'injection intra-veineuse est de 200 à 500 mg de chlorure de tétraéthylammonium sans excéder 7 mg par kg de poids corporel du récepteur.

6. Utilisation selon la revendication 4, dans laquelle le dosage pour l'injection intra-musculaire est de 1000 à 1200 mg de chlorure de tétraéthylammonium sans excéder 20 mg par kg de poids corporel du récepteur.